# EUROPEAN PATENT APPLICATION

(11) **EP 2 713 182 A2**
(43) Date of publication of application: **02.04.2014**
(21) Application number: 13184061.3
(22) Date of filing: 12.09.2013
(51) Int. Cl.: G01T 1/29

(54) **Radiography imaging and radiation detection system**

(30) Priority: 28.09.2012 KR 20120108932
(71) Applicant: Samsung Electronics Co., Ltd, Gyeonggi-do 443-742 (KR)
(72) Inventor: Yang, Soo Sang, 313-2002 Gyeonggi-do (KR); Park, Sung Kyu, 105-1504 Gyeonggi-do (KR); Choi, Byung Sun, 436-402 Gyeonggi-do (KR)
(74) Representative: Lubberdink, Pim

(57) **Abstract**

A radiography imaging apparatus comprises a radiation source to generate radiation for irradiation of a patient. A radiation detection unit comprises a plurality of detection modules (125) to detect radiation having passed through the patient and to convert the radiation into an electrical signal and to convert the electrical signal into a digital signal to provide digital data. A data collection unit collects the digital data from the plurality of detection modules (125) for use in generation of a radiography image of the patient, wherein each of the plurality of detection modules (125) transmits the digital data to a neighboring detection module, and at least one of the plurality of detection modules (125) transmits cumulative digital data acquired from the plurality of detection modules to the data collection unit.

## Description

### FIELD OF THE INVENTION

The present invention relates to a radiation detection, radiography imaging and image reconstruction system.

### BACKGROUND

A radiography imaging apparatus irradiates a target object with radiation and analyzes radiation having passed through the target object to examine an internal structure of the target object. Permeability differs according to tissues constituting the target object, and thus, the internal structure of the target object may have an attenuation coefficient representing permeability as a numeric value. A radiography imaging apparatus may comprise a radiography imaging apparatus transmitting X-ray radiation using a rotable C-arm, for example, having a radiation detector and emitter located at opposite ends of the C-arm rotatable around a patient or may comprise a relatively complex computed tomography (CT) scanning apparatus for omni-directional transmission of X-ray radiation around the patient. The systems reconstruct an image using a computer. The CT apparatus may be referred to as a computer tomography apparatus, a computerized tomography apparatus, or the like.

A radiation detection unit of the CT apparatus is configured in such a way that a plurality of detection modules is arranged in array form and are connected to a back plane (BP) board to collect data through a cable to transmit X-ray data, for example. Multiple (e.g. tens of) detection modules may be included in a radiation detection unit so that when each detection module is connected to the BP board through a cable, the size of the X-ray detection unit may be increased and contact error between cables may occur due to the structure of densely arranged detection modules. A system according to invention principles addresses these deficiencies and related problems.

### SUMMARY

A system according to invention principles provides a radiography imaging system having a plurality of detection modules sequentially transmitting data where individual detection modules communicate with a back plane (BP) board in a wireless manner advantageously reducing the size of a radiation detection unit and preventing connector contact error.

A radiography imaging apparatus comprises a radiation source to generate radiation for irradiation of a patient. A radiation detection unit comprises a plurality of detection modules to detect radiation having passed through the patient and to convert the radiation into an electrical signal and to convert the electrical signal into a digital signal to provide digital data. A data collection unit collects the digital data from the plurality of detection modules for use in generation of a radiography image of the patient, wherein each of the plurality of detection modules transmits the digital data to a neighboring detection module, and at least one of the plurality of detection modules transmits cumulative digital data acquired from the plurality of detection modules to the data collection unit.

In a feature of the invention each of the plurality of detection modules transmits the digital data to a neighboring adjacent detection module and the radiation source and the radiation detection unit are installed in a gantry rotated about the patient. The data collection unit is formed on a back plane (BP) board of a frame with the plurality of detection modules installed thereon and each of the plurality of detection modules transmits the digital data to a neighboring detection module in a single direction using a wired communication method. In one embodiment the wired communication method is performed through a cable installed in a frame with the plurality of detection modules installed therein. A last detection module, receiving digital data, transmits the received digital data and digital data acquired by the last detection module, comprising the cumulative digital data, to the data collection unit using a wired communication method.

In another embodiment, the detection modules transmit the digital data to a neighboring adjacent detection module in a single direction using a wireless communication method. A last detection module, receiving digital data, transmits the received digital data and digital data acquired by the last detection module, comprising the cumulative digital data, to the data collection unit using a wireless communication method. The wireless communication method uses at least one of, Zigbee, wireless fidelity (Wi-Fi), radio frequency identification (RFID), Bluetooth, and near field communication (NFC). Further, the plurality of detection modules receives power through a power cable installed in the frame.

In another feature of the invention, a plurality of detection modules detect radiation having passed through a patient, convert the radiation into an electrical signal and convert the electrical signal into a digital signal to provide digital data. The data collection unit collects the digital data from the plurality of detection modules for use in generation of a radiography image of the patient. Each of the plurality of detection modules transmits the digital data to a neighboring adjacent detection module, and at least one of the plurality of detection modules transmits cumulative digital data acquired from the plurality of detection modules to the data collection unit.

In a further feature of the invention, a radiography imaging apparatus comprises a radiation source to generate radiation for irradiation of a patient. A radiation detection unit comprises a plurality of detection modules to detect radiation having passed through the patient to convert the radiation into an electrical signal and to convert the electrical signal into a digital signal to provide digital data. A data collection unit collects the digital data from the plurality of detection modules for use in generation of a radiography image of the patient wherein each of the plurality of detection modules transmits the digital data, to provide cumulative digital data acquired from the plurality of detection modules, to the data collection unit using a wireless communication method.

In yet another feature of the invention, a method of controlling a radiography imaging apparatus comprising a plurality of detection modules to detect radiation and a data collection unit to receive data from the plurality of detection modules, comprises irradiating a patient with radiation. The method uses a plurality of detection modules to detect radiation having passed through the patient, converts the detected radiation into an electrical signal and converts the electrical signal into a digital signal to provide digital data. The method transmits the digital data between neighboring adjacent detection modules of a plurality of detection modules, in a single direction and transmits cumulative digital data acquired from the plurality of detection modules to a data collection unit.

### BRIEF DESCRIPTION OF THE DRAWINGS

These and/or other aspects of the invention will become apparent and more readily appreciated from the following description of the embodiments, taken in conjunction with the accompanying drawings of which:
FIG. 1 shows a control block diagram of a computed tomography apparatus according to invention principles;
FIG. 2A shows diagram showing an overall structure of a computed tomography apparatus according to invention principles;
FIG. 2B shows a transverse cross sectional view of a gantry of a computed tomography apparatus according to invention principles;
FIG. 3A shows a schematic exploded perspective view of an internal structure of an X-ray detection unit;
FIG. 3B shows a perspective view of a structure of a detection module;
FIG. 3C shows a schematic diagram showing a structure of an analog-to-digital conversion module;
FIG. 4 shows a diagram showing a connection relationship between a detection module and a data collection unit in a conventional X-ray detection unit;
FIG. 5 shows a control block diagram of the X-ray detection unit of a computed tomography apparatus according to invention principles;
FIGS. 6A through 6D are diagrams showing examples of a communication method of communication units of detection modules;
FIG. 7 shows a control block diagram of an X-ray detection unit of a computed tomography apparatus according to invention principles;
FIG. 8 shows a schematic exploded perspective view of an internal structure of an X-ray detection unit of a computed tomography apparatus according to invention principles;
FIG. 9 shows a diagram showing an example of a power supply method which is applicable to an X-ray detection unit of a computed tomography apparatus according to invention principles;
FIG. 10 shows a flowchart of a method of controlling a computed tomography apparatus according to invention principles; and
FIG. 11 shows a flowchart of a method of controlling a computed tomography apparatus according to invention principles.

### DETAILED DESCRIPTION

Hereinafter, a radiography imaging apparatus is described in detail with regard to an embodiment of the present invention with reference to the accompanying drawings.

Radiation is a combination of energy in the form of particles or electromagnetic waves, which is emitted when an unstable radiation nuclide is converted into a more stable nuclide. A representative example of such radiation may include infrared rays and visible rays as well as X-rays, ultrasound waves, alpha rays, beta rays, gamma rays, and neutron rays. As used herein, for convenience of description, radiation comprises X-ray radiation, but the embodiments of the present invention are not limited thereto. An X-ray imaging apparatus is usable for intra-oral X-ray imaging and mammography for breast imaging, for example, by acquiring an image at an angle or images at various angles during a scan by a CT scan unit. A CT scan two dimensional (2D) image or a three dimensional (3D) image data set may be derived by reconstruction and combination of image data acquired at one or more angles.

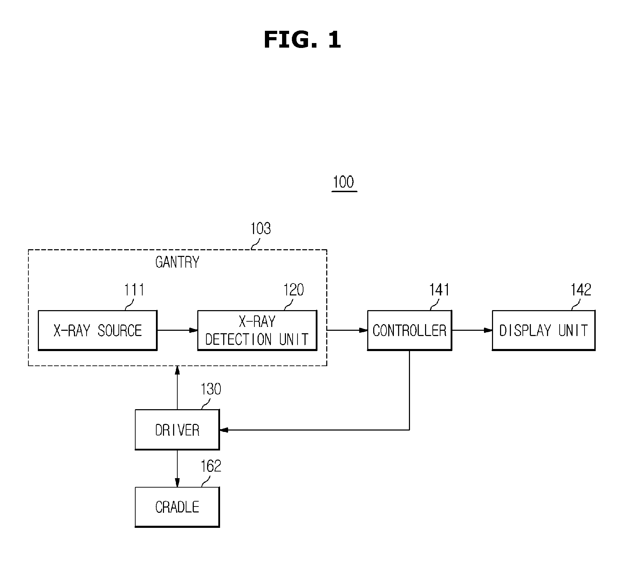

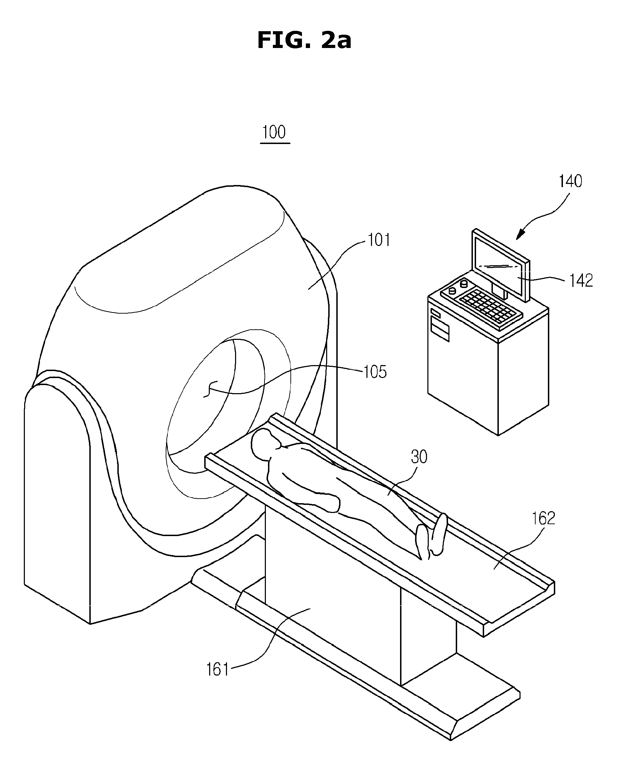

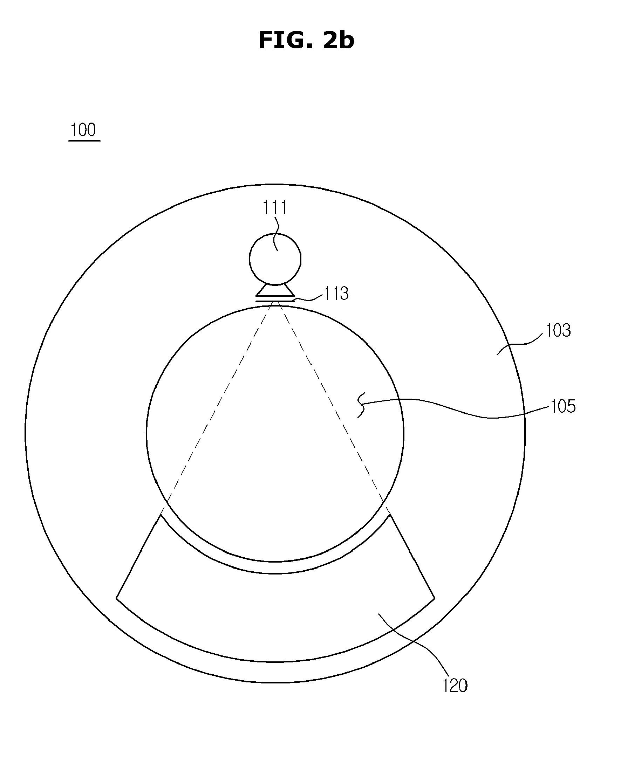

FIG. 1 shows a computed tomography apparatus 100, FIG. 2A shows an overall structure of a computed tomography apparatus 100 of FIG. 1, and FIG. 2B shows a transverse cross sectional view of a gantry 103 of the computed tomography apparatus 100 of FIG. 1. Hereinafter, the structure and operation of the computed tomography apparatus 100 is described with reference to FIGS. 1, 2A, and 2B. Computed tomography apparatus 100 includes an X-ray source 111 generating X-rays and irradiating a target object or patient, an X-ray detection unit 120 to detect X-rays having passed through the target patient and acquire X-ray data, a controller 141 reconstructing the acquired X-ray data and generating an image of the target patient, a driver 130 driving the gantry 103 and a cradle 162, and a display unit 142 to display the generated image of the target object or patient. The X-ray source 111 and the X-ray detection unit 120 are installed in the gantry 103, irradiate the target object or patient with the X-rays and detect the X-rays having passed through the target patient while rotating about the target patient to predetermined angles.

The X-ray source 111 includes an X-ray tube to generate X-rays generated in response to power received from an external power supply (not shown). When a high voltage is applied between a cathode and an anode of the X-ray tube, thermions are accelerated and collide with a target material of the anode to generate the X-rays. A generator generating the high voltage may be installed inside or outside the gantry 103. Energy of the X-rays is adjustable according to a tube voltage applied to the X-ray source 111 and intensity or dose of the X-rays is adjustable according to the tube current and a desired X-ray exposure time. The energy, intensity, or dose of the X-rays may be determined according to the type or thickness of the target patient, a diagnosis purpose, or the like.

The X-ray source 111 may generate monochromatic X-rays or polychromatic X-rays. When the X-ray source 111 generates polychromatic X-rays having a specific energy band, the energy band of the irradiated X-rays may be defined by an upper limit and a lower limit. The upper limit of the energy band, that is, maximum energy of the irradiated X-rays is adjustable according to the amplitude of the tube voltage. The lower limit of the energy band, that is, minimum energy of the irradiated X-rays is adjustable according to a filter. The filter passes or filters X-rays in a specific energy band. A filter for filtering X-rays in a low energy band may be installed in X-ray generator 111 (or elsewhere) having an adjustable lower energy band limit that may be raised increasing average energy of X-rays, for example.

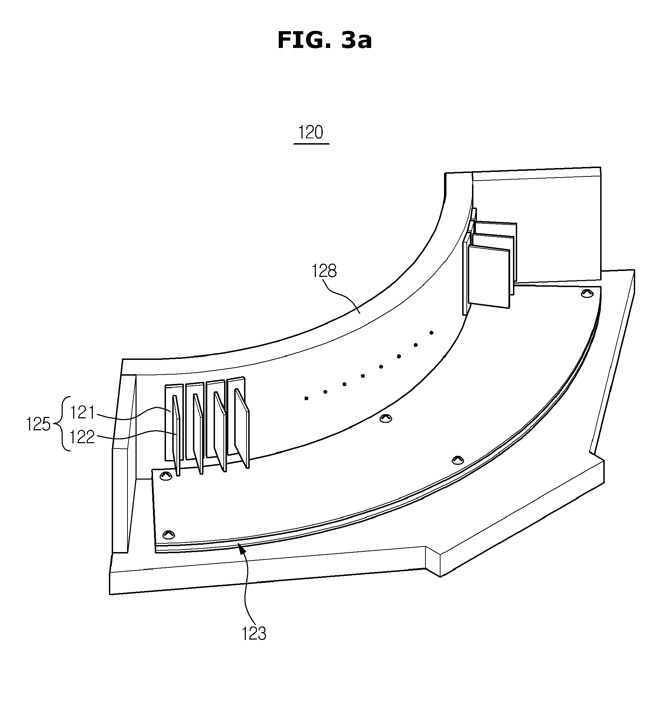

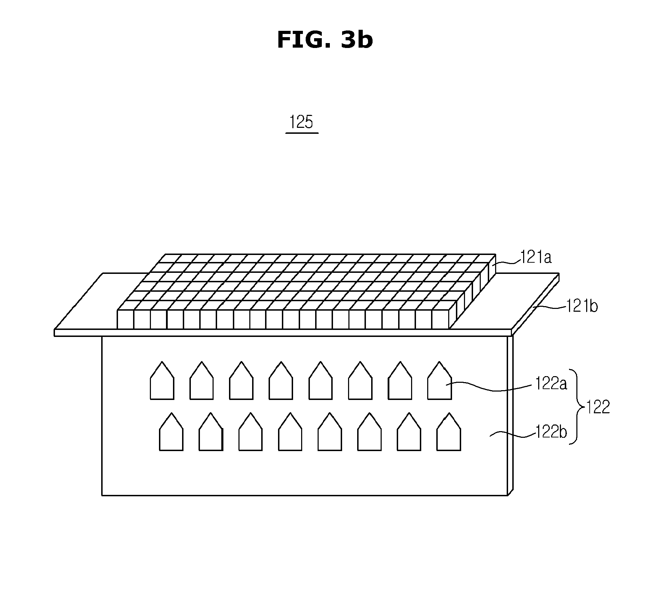

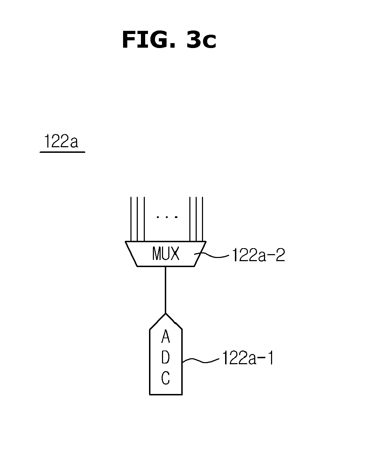

The X-ray detection unit 120 includes a plurality of detection modules arranged in an array form. An individual detection module detects X-rays having passed through the target patient, converts the detected X-rays into an electrical signal to acquire digital X-ray data, and transmits the digital X-ray data to the controller 141. The X-ray detection unit 120 is described in detail with reference to FIG. 3. The controller 141 reconstructs an image using the digital X-ray data transmitted from the X-ray detection unit 120. Image reconstruction employs a known method such as an iterative method, a direct Fourier method or a filtered back projection, for example. A reconstructed image is output to the display unit 142. The driver 130 includes a driving motor to drive the gantry 103 and a driving motor to drive the cradle 162. The controller 141 directs the driver 130 in controlling rotation of the gantry 103 and movement of the cradle 162.

Referring to FIGS. 2A and 2B, the computed tomography apparatus 100 includes a housing 101 that includes and supports the X-ray source 111 and X-ray detection unit 120. , The cradle (patient support table) 162 conveys a target patient 30, unit 161 supports the cradle 162, and a workstation 140 displays an image of the target patient 30 and receive control commands for operation of the computed tomography apparatus 100 from a user. The workstation 140 includes the display unit 142 and may also in one embodiment incorporate controller 141. The gantry 103 is installed in the housing 101, and the X-ray source 111 and the X-ray detection unit 120 are installed in the gantry 103. When the target patient 30 lies on the cradle 162, the driver 130 moves the cradle 162 to a bore 105 formed in a central portion of the housing 101 so that the target patient 30 is moved in the cradle 162. The controller 141 controls the driver 130 to adjust a transfer distance of the cradle 162 such that an examination portion of the target patient 30 to be imaged is positioned in the bore 105.

The X-ray source 111 and X-ray detection unit 120 installed in the gantry 103 are fixed opposite to each other such that the X-rays irradiated from the X-ray source 111 are detected by the X-ray detection unit 120. In response to initiation of computed tomography, the driver 130 provides rotation force to the gantry 103. X-rays pass through target patient 30 from the X-ray source 111 while the gantry 103 rotates about the bore 105 and are detected by X-ray detection unit 120. The controller 141 controls rotation speed and rotation number of the gantry 103 through the driver 130. A collimator 113 is installed on a front surface of the X-ray source 111, and is used to adjust the width of X-ray beam radiated from the X-ray source 111. Thus, the collimator 113 reduces scattering rays to reduce over exposure of the target patient 30. In addition, although not shown, a collimator may also be installed on a front surface of the X-ray detection unit 120, to detect X-rays limited to a specific region of interest. The collimator installed on the front surface of the X-ray detection unit 120 removes scattered X-rays and adjusts the width of the detected X-ray beam to determine the thickness of a slice.

FIG. 3A shows a schematic exploded perspective view of an internal structure of the X-ray detection unit 120, FIG. 3B shows a perspective view of a structure of a detection module 125, and FIG. 3C shows a structure of an analog-to-digital conversion module. The X-ray detection unit 120 comprises a plurality of detection modules 125 installed on a frame 128. For example, 40 to 60 detection modules 125 may be installed in a one-dimensional array form on the frame 128. Individual detection modules 125 include a detector 121 for detecting incident X-rays and a data acquisition unit 122 to acquire resultant digital X-ray data. The detection module 125 is configured with detector 121 installed on an upper portion of the frame 128, on which the X-rays are incident, as shown in FIG. 3A. Hereinafter, for convenience of description, a portion on which X-rays are incident is referred to as an upper portion.

Referring to FIG. 3B, the detection module 125 includes the detector 121 including a substrate 121b and light receiving devices 121a formed thereon, and the data acquisition unit 122 to acquire digital X-ray data. Although not shown, the detector 121 and the data acquisition unit 122 may be connected through a flexible cable. As described above, a collimator (not shown) may be installed on the surface of the light receiving devices 121a so as to detect X-rays with respect to a specific limited region of interest. When X-rays are incident on the light receiving devices 121a, the light receiving devices 121a generate electric charges responsive to energy and dose of the incident X-rays. The light receiving devices 121a may comprise photo diodes, for example. As shown in FIG. 3B, a plurality of light receiving devices may be arranged in a two-dimensional array form such that each light receiving device functions as one pixel. The light receiving devices 121a may directly detect X-rays in one method and may detect visible rays converted from the X-rays in another method. The former method is referred to as a direct conversion method and the latter method is referred to as an indirect conversion method. In the indirect conversion method, a scintillator is disposed on a front surface of the light receiving devices 121a and converts X-rays into visible rays. The scintillator comprises, for example, a film type GADOX scintillator, a micro column type or needle structured type CSI (T1) unit, for example.

In addition, the light receiving devices 121a may be formed of materials to detect visible rays, such as a-Si or the like. In the direct conversion method, the light receiving devices 121a may be formed of materials that directly detect X-rays, such as a-Se, CdZnTe, Hgl₂, Pbl₂, or the like.

A read-out circuit is formed on the substrate 121b so as to read the electric charges generated by the light receiving devices 121a as an electrical signal such as a voltage signal or a current signal and to input the electrical signal to the data acquisition unit 122. The data acquisition unit 122 includes a substrate 122b and analog-to-digital conversion modules 122a formed thereon. When the electrical signal is input to the data acquisition unit 122 from the detector 121, the analog-to-digital conversion modules 122a convert the analog electrical signal into a digital electrical signal to provide digital X-ray image representative data. The data acquisition unit 122 is also referred to as a data acquisition system (DAS).

Individual analog-to-digital conversion modules 122a include a plurality of channels, and one analog-to-digital conversion module 122a receives the electrical signal from a plurality of pixels of the detector 121 and converts the electrical signal into a digital signal. To this end, as shown in FIG. 3C, the analog-to-digital conversion module 122a includes an analog-to-digital converter (ADC) 122a-1 and a multiplexer 122a-2 having a plurality of channels connected to the ADC 122a-1. Electrical signals for respective pixels, output from the detector 121, are input to the ADC 122a-1 through the multiplexer 122a-2 and are converted into digital signals. Electrical signals output from the detector 121 are relatively small signals, and thus, an amplifier is installed at an input terminal of the multiplexer 122a-2 such that the electrical signals are amplified to exceed a predetermined size and converted into digital signals.

Referring back to FIG. 3A, the X-ray detection unit 120 includes a data collection unit 123 to collect data and the data collection unit 123 is formed on a back plane (BP) board of the frame 128. The data collection unit 123 collects the digital X-ray data from each detection module 125 and transmits the digital X-ray data to the controller 141 installed outside the gantry 103 or in the workstation 140, and the controller 141 reconstructs the image of the target patient 30 using the transmitted digital X-ray data.

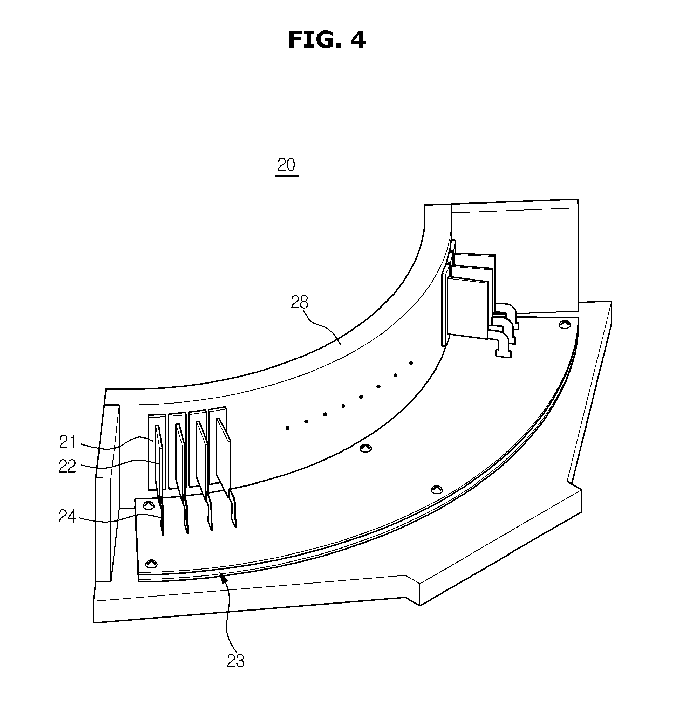

FIG. 4 shows a connection relationship between a detection module 25 and a data collection unit 23 in a known X-ray detection unit 20. Known detection modules 25 and the data collection unit 23 are connected through cables 24 in the X-ray detection unit 20, thus occupying a large space, and being vulnerable to contact error exacerbated by a dense arrangement of the detection modules 25. A computed tomography apparatus advantageously employs a plurality of detection modules 125 (FIG. 3A) that sequentially transmit data with the last detection module 125 being exclusively connected to the data collection unit 123, thereby reducing the size of the X-ray detection unit 120 and reducing contact error between cables.

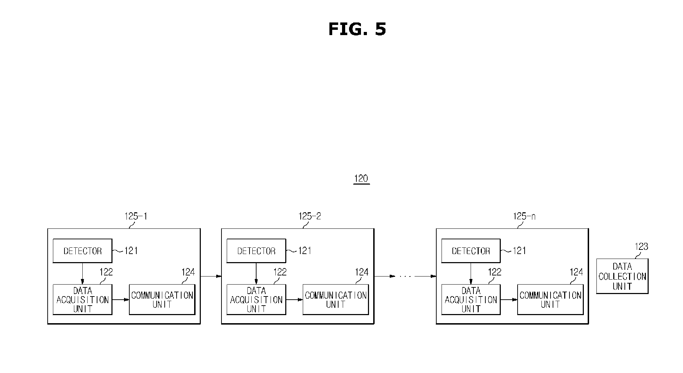

FIG. 5 shows a control block diagram of the X-ray detection unit 120 of a computed tomography apparatus. X-ray detection unit 120 includes the detector 121 to detect X-rays having passed through a target patient and to convert the X-rays into an electrical signal. The data acquisition unit 122 to converts the analog electrical signal into a digital signal to provide digital X-ray data, and a plurality of detection modules 125 each including a communication unit 124, transmit and receive data to and from a neighboring detection module.

The X-ray detection unit 120 includes n (≥3) detection modules 125. The plurality of detection modules 125 transmit the digital X-ray data in a particular direction, in one embodiment. Specifically, a detection module receiving the digital X-ray data retransmits the received digital X-ray data and digital X-ray data acquired by a data acquisition unit of the corresponding detection module to an adjacent detection module so the digital X-ray data is sequentially and cumulatively transmitted.

However, it is not necessary to cumulatively transmit the digital X-ray data. For example, one of the detection modules 125 having received data from the previous detection module 125 may instantly transmit the data to the next detection module 125 without waiting for the data to be cumulated with another data. Also, the last detection module 125, right after receiving data from an adjacent detection module 125, also transmits the data to the data acquisition unit 122 without waiting for respective pieces of data detected by all the other detection modules 125.

Individual units of the plurality of detection modules 125 are advantageously connected to each other in a daisy chain form and the communication unit 124 includes a buffer to temporarily or non-temporarily store data.

As shown in FIG. 5, when the X-ray detection unit 120 includes the n detection modules 125, a first detection module 125-1 transmits digital X-ray data acquired by the data acquisition unit 122 to a second detection module 125-2 through the communication unit 124. The digital X-ray data is transmitted up to an nₜₕ detection module 125-n using the same method. In addition, the communication unit 124 of the nₜₕ detection module 125-n transmits digital X-ray data acquired by the first detection module 125-1 to digital X-ray data acquired by the nₜₕ detection module 125-n, to the data collection unit 123. Here, the communication units 124 of detection modules 125-1 to 125-n transmit and receive data using a wired or wireless method.

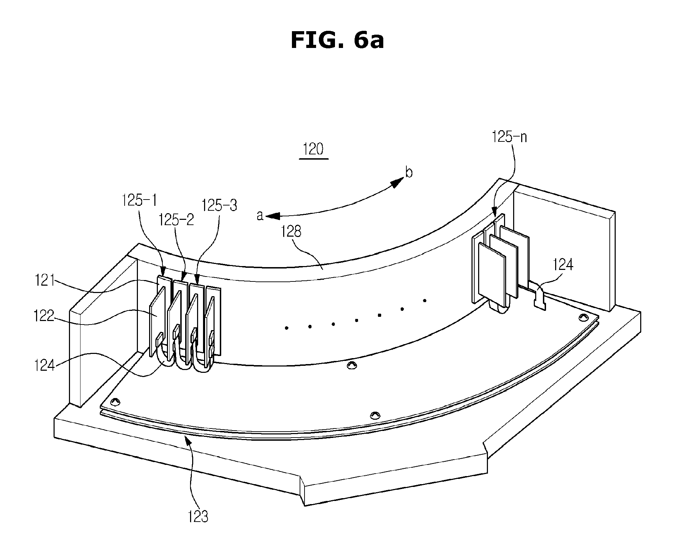

FIGS. 6A through 6D show communication systems of communication units 124 of the detection modules 125. Referring to an example shown in FIG. 6A, the communication unit 124 includes a cable supporting communication between neighboring adjacent detection modules 125. In this case, a communication direction may be determined as one direction. In the example shown in FIG. 6A, data is sequentially transmitted in a direction from 'a' to 'b', or alternatively, may be transmitted in a direction 'b' to 'a'. The first detection module 125-1 transmits the digital X-ray data acquired by the data acquisition unit 122 to the second detection module 125-2 through the cable and the second detection module 125-2 transmits digital X-ray data acquired by a data acquisition unit thereof and the digital X-ray data received from the first detection module 125-1 to a third detection module 125-3 through the cable (124). Data is transmitted up to the nₜₕ detection module 125-n using the same method.

The nₜₕ detection module 125-n that lastly receives data, transmits accumulated data to the data collection unit 123. In this case, as shown in FIG. 6A, the communication unit 124 of the nₜₕ detection module 125-n includes the cable so as to transmit data to the data collection unit 123 using a wired communication method or to transmit data to the data collection unit 123 using a wireless communication method. That is, the communication unit 124 of the nₜₕ detection module 125-n may transmit and receive data using a wired method or may receive data using a wired method and transmit data using a wireless method.

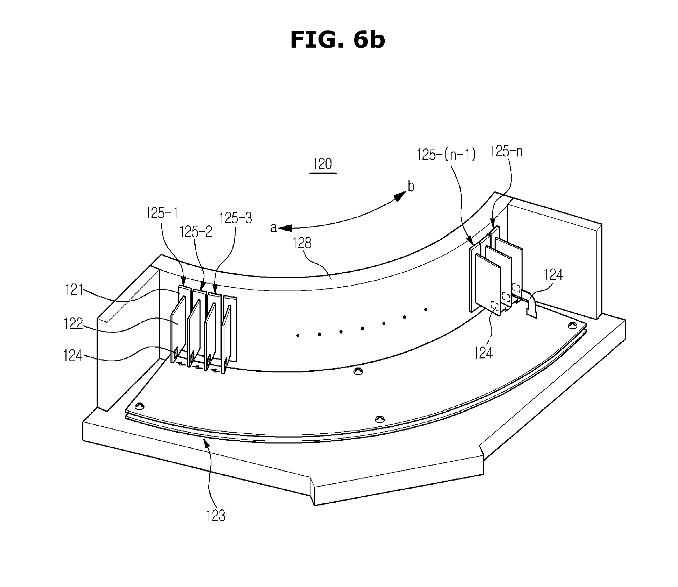

Referring to another example shown in FIG. 6B, the communication unit 124 performs communication using a wireless communication method such that data is transmitted and received between neighboring adjacent detection modules 125 without a cable using a wireless method. The communication unit 124 performs communication using at least one of different wireless communication methods including Zigbee, wireless fidelity (Wi-Fi), radio frequency identification (RFID), Bluetooth, near field communication (NFC), infrared communication, for example. Other communication methods as well as the wireless communication methods may be applied to the communication unit 124.

Thus, digital X-ray data is transmitted from the first detection module 125-1 to the nₜₕ detection module 125-n using a wireless communication method. The nₜₕ detection module 125-n receives the cumulative digital X-ray data acquired from the modules from the first detection module 125-1 to the nₜₕ detection module 125-n and transmits the received cumulative digital X-ray data to the data collection unit 123. In this case, the communication unit 124 of the nₜₕ detection module 125-n transmits data to the data collection unit 123 through the cable (124) using a wired method, as shown in FIG. 6B, or may transmit data to the data collection unit 123 using a wireless method as in the other detection modules. That is, the communication unit 124 of the nₜₕ detection module 125-n may receive data using a wireless method and transmit data using a wired method, or may transmit and receive data using a wireless method.

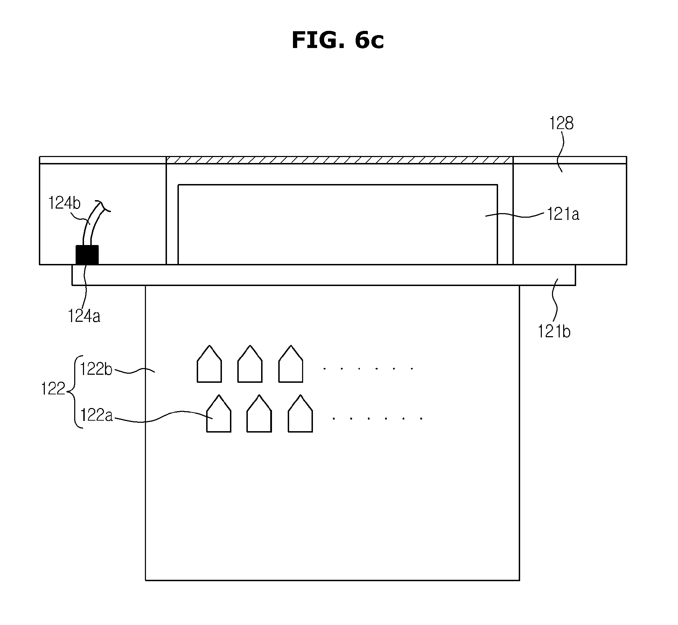

In another example shown in FIG. 6C, the plurality of detection modules 125 transmit and receive data through a cable 124b. Here, the cable 124b to transmit data may be installed in the frame 128. The plurality of detection modules 125 may be fixed to the frame 128. In this regard, the cable 124b to transmit data is installed in the frame 128 and a connection terminal 124a is installed on the substrate 121b of the detector 121, and thus, the connection terminal 124a and the cable 124b are connected to each other so as to transmit digital X-ray data through the cable 124b when the detection modules 125 are fixed to the frame 128.

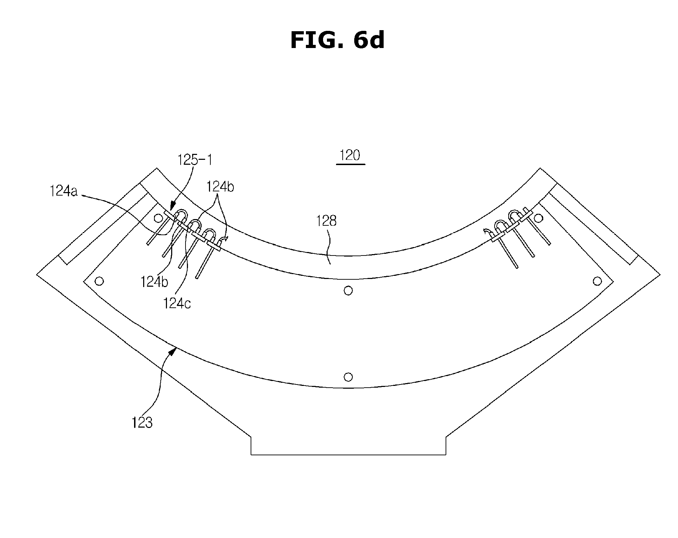

FIG. 6D shows a connection relationship between the plurality of detection modules 125 of the X-ray detection unit 120 shown in FIG. 6C. FIG. 6D shows a front view of the X-ray detection unit 120, in which a front surface of the frame is removed. Connection terminal 124a transmits data and is formed on the first detection module 125-1 connected to the cable 124b. In addition, the cable 124b is connected to a connection terminal 124c to receive data and is formed on the second detection module 125-2, such that data transmitted from the first detection module 125-1 is transmitted to the second detection module 125-2. Moreover, the connection terminal 124b and the connection terminal 124c formed on the third detection module 125-3, are connected to each other through the cable 124b, and data from the first and second detection modules 125-1 and 125-2 is transmitted to the third detection module 125-3 through the cable 124b. Cumulative data is transmitted up to the nₜₕ detection module 125-n. Thus, the second detection module 125-2 to a (n-1)ₜₕ detection module 125-(n-1) each include a connection terminal to transmit data and a connection terminal to receive data. In addition, a communication unit 124-n of the nₜₕ detection module 125-n transmits cumulative data acquired from first to the nₜₕ detection module 125-n, to the data collection unit 123 using a wired or wireless communication method.

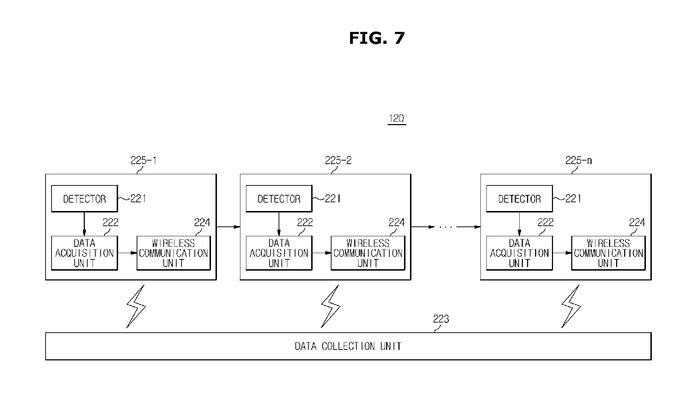

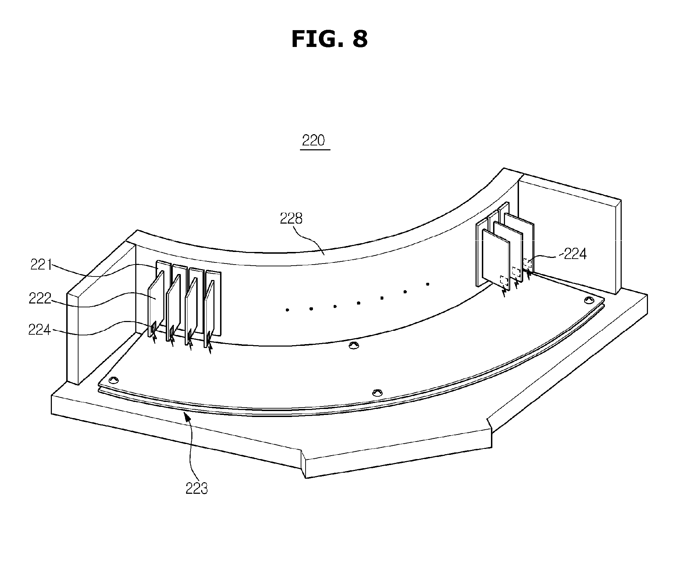

FIG. 7 shows an X-ray detection unit 120 of a computed tomography apparatus 100 and FIG. 8 shows an exploded perspective view of an internal structure of the X-ray detection unit 120 of the computed tomography apparatus 100. The control block diagram of FIG. 7 shows X-ray detection unit 120, and thus, the remaining elements of the computed tomography apparatus 100 are the same as in FIGS. 1 through 4. Referring to FIGS. 7 and 8, each of a plurality of detection modules 225 includes a detector 221 to detect X-rays having passed through a target patient and to convert the X-rays into an electrical signal. A data acquisition unit 222 converts the analog electrical signal into a digital signal to provide digital X-ray data, and a wireless communication unit 224 transmits the digital X-ray data to a data collection unit 223 using a wireless communication method. X-ray detection unit 120 includes n(≥3) detection modules 125.

Although not shown in FIG. 8, the structure of the detection module 125 shown in FIG. 3B is applied to the detection modules 225. The data acquisition unit 222 includes a substrate and a plurality of digital conversion units formed thereon. The digital conversion units each include a plurality of channels, and thus, an electrical signal per pixel is input to each channel. The analog electrical signal converted by the detector 221 is input to a digital conversion unit of the data acquisition unit 222 and is converted into a digital signal. The wireless communication unit 224 is implemented as a wireless communication module to transmit and receive data using a wireless method and is formed on a substrate of the data acquisition unit 222 (FIG. 8). The wireless communication unit 224 transmits digital X-ray data acquired by the data acquisition unit 222 to the data collection unit 223. The wireless communication unit 224 performs communication using at least one of different wireless communication methods including, Zigbee, Wi-Fi, RFID, Bluetooth, NFC, infrared communication, for example.

The data collection unit 223 receives digital X-ray data from each of the plurality of detection modules 225 using a wireless communication method. The received digital X-ray data is transmitted to the controller 141 (FIG. 141) installed outside the gantry 103 or in the workstation 140 to reconstruct an image.

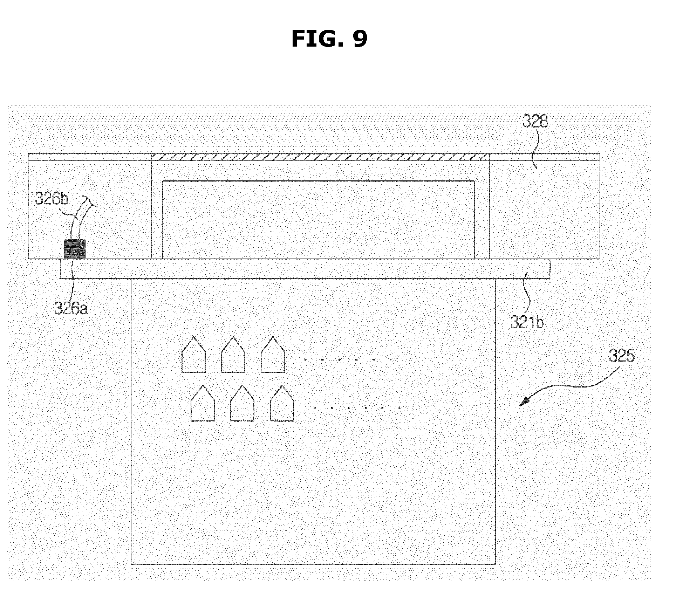

FIG. 9 shows a power supply used to power X-ray detection unit 221 of a computed tomography apparatus. A power input terminal 326a is formed on a substrate 321b of the X-ray detection unit 321 and a power cable 326b is installed in a frame 328. When a detection module 225 is fixed to the frame 328, the power input terminal 326a may be inserted into the frame 328 and may be connected to the power cable 326b installed in the frame 328 so as to receive power from the power cable 326b. A structure for fixing each detection module 225 to the frame 228 is the same as in FIGS. 6C and 6D. The power supply structure advantageously reduces space restriction limitations due to volume of power cables or contacts with other cables. Accordingly, the power supply method shown in FIG. 9 may be applied to the previously described computed tomography systems.

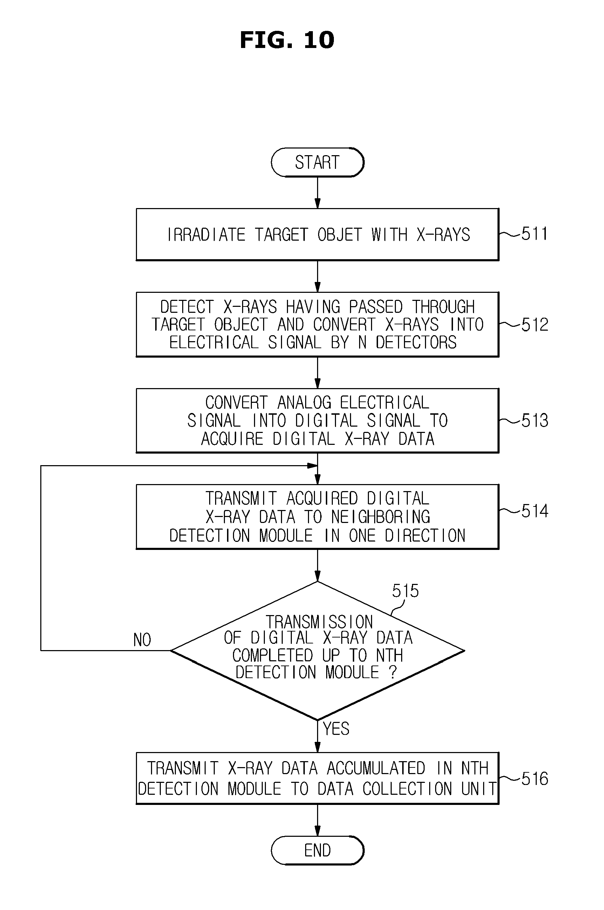

FIG. 10 is a flowchart of a method of controlling a computed tomography apparatus having an X-ray detection unit including n detection modules which each include a detector and a data acquisition unit. A target patient (511) is irradiated and X-rays having passed through the target patient are detected and are converted into electrical signals by n detectors (512). The converted electrical signal is an analog signal such as a voltage signal, a current signal, or the like. While the X-rays are generated and detected, a gantry may be rotated about the target patient, and an X-ray source and X-ray detection unit installed in the gantry may be rotated together. The electrical signal converted by the detector is input to a data acquisition unit DAS connected to each detector. The data acquisition unit DAS converts the analog electrical signal into a digital signal to provide digital X-ray data (513).

In addition, the acquired digital X-ray data is transmitted to a neighboring detection module in one direction (514) where the n detection modules are connected to each other in a daisy chain form. A first detection module transmits digital X-ray data to a second adjacent detection module, and the second detection module transmits digital X-ray data acquired by the second detection module together with the digital X-ray data received from the first detection module, to an adjacent third detection module. The digital X-ray data is transmitted up to an nₜₕ detection module in this manner. In order to transmit digital X-ray data to a neighboring detection module, a wired communication method or a wireless communication method may be used. As previously described with regard to the computed tomography apparatuses, neighboring detection modules may be connected to each other through a flexible cable, a wireless communication module may be installed in each detection module so as to receive or transmit data from a neighboring detection module using a wireless method, or a cable may be installed in a frame of an X-ray detection unit such that a connection terminal and the cable are connected to each other so as to transmit and receive data when each detection module is installed on the frame. When transmission of digital X-ray data is completed up to the nₜₕ detection module (YES of 515), digital X-ray data accumulated in the nₜₕ detection module is transmitted to a data collection unit (516). The data collection unit is formed on a BP board of the X-ray detection unit and transmits collected digital X-ray data to a controller installed outside a gantry or in a workstation to perform image reconstruction or the like.

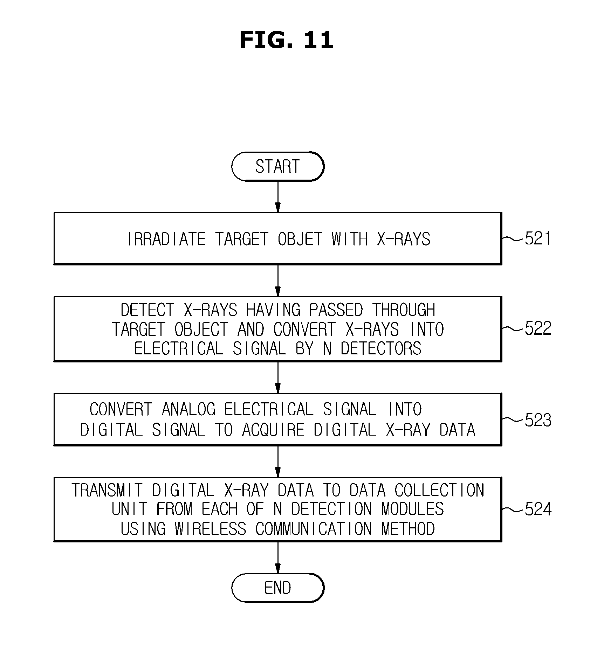

FIG. 11 shows a flowchart of a method of controlling a computed tomography apparatus including n detection modules individually having a detector and a data acquisition unit. X-rays are directed to a target patient (521) and resultant X-rays having passed through a target patient are detected and are converted into an electrical signal by n detectors (522). The electrical signal is input to a data acquisition unit DAS connected to each detector and converted into a digital signal to provide digital X-ray data (523). In addition, the digital X-ray data is transmitted from the n detection modules to the data acquisition unit using a wireless communication method (524). Data is transmitted to the data acquisition unit using at least one of different wireless communication methods including, Zigbee, Wi-Fi, RFID, Bluetooth, NFC, for example.

As is apparent from the above description, in a radiography imaging apparatus and a method of controlling the same, a plurality of detection modules may sequentially transmit data or each detection module may communicate with a BP board using a wireless method, thereby reducing the size of a radiation detection unit and preventing contact error of connectors. Although a few embodiments of the present invention have been shown and described, it would be appreciated by those skilled in the art that changes may be made in these embodiments without departing from the principles and spirit of the invention, the scope of which is defined in the claims and their equivalents.

The above-described embodiments can be implemented in hardware, firmware or via the execution of software or computer code that can be stored in a recording medium such as a CD ROM, an RAM, a floppy disk, a hard disk, or a magneto-optical disk or computer code downloaded over a network originally stored on a remote recording medium or a non-transitory machine readable medium and to be stored on a local recording medium, so that the methods described herein can be rendered via such software that is stored on the recording medium using a general purpose computer, or a special processor or in programmable or dedicated hardware, such as an ASIC or FPGA. As would be understood in the art, the computer, the processor, microprocessor controller or the programmable hardware include memory components, e.g., RAM, ROM, Flash, etc. that may store or receive software or computer code that when accessed and executed by the computer, processor or hardware implement the processing methods described herein. In addition, it would be recognized that when a general purpose computer accesses code for implementing the processing shown herein, the execution of the code transforms the general purpose computer into a special purpose computer for executing the processing shown herein. The functions and process steps herein may be performed automatically or wholly or partially in response to user command. An activity (including a step) performed automatically is performed in response to executable instruction or device operation without user direct initiation of the activity. No claim element herein is to be construed under the provisions of 35 U.S.C. 112, sixth paragraph, unless the element is expressly recited using the phrase "means for."

## Claims

1. A radiography imaging apparatus comprising:
a radiation source (111) to generate radiation and for irradiation of a target object; and
a radiation detection unit (120) comprising a plurality of detection modules (25, 125, 225) to detect radiation having passed through the target object and to convert the radiation into an electrical signal and to convert the electrical signal into a digital signal to provide digital data and a data collection unit (23, 123, 223) to collect the digital data from the plurality of detection modules,
**characterized in that** each of the plurality of detection modules (25, 125, 225) transmits the digital data to a neighboring detection module, and at least one of the plurality of detection modules transmits digital data transmitted from the neighboring detection module to the data collection unit (23, 123, 223).

2. The radiography imaging apparatus according to claim 1, **characterized in that**:
the radiation detection unit (120) are installed in a gantry rotated about the target object;
the data collection unit (23, 123, 223) is formed on a back plane (BP) board of a frame with the plurality of detection modules (25, 125, 225) installed thereon; and
the plurality of detection modules (25, 125, 225) receives power through a power cable installed in the frame.

3. The radiography imaging apparatus according to claim 1, **characterized in that**:
each of the plurality of detection modules (25, 125, 225) transmits the digital data to a neighboring detection module in a single direction using a wired communication method; and
the wired communication method is performed through a cable (24) installed in a frame with the plurality of detection modules (25, 125, 225) installed therein.

4. The radiography imaging apparatus according to claim 1, **characterized in that** each of the plurality of detection modules (25, 125, 225) transmits the digital data to a neighboring detection module in a single direction using a wireless communication method.

5. The radiography imaging apparatus according to any one of claims 3 and 4, wherein a last detection module, lastly receiving digital data, transmits the received digital data and digital data acquired by the last detection module to the data collection unit using a wired communication method or a wireless communication method.

6. A radiation detection unit comprising:
a plurality of detection modules (25, 125, 225) to detect radiation having passed through a target object to convert the radiation into an electrical signal and to convert the electrical signal into a digital signal to acquire digital data; and
a data collection unit (23, 123, 223) to collect the digital data from the plurality of detection modules,
**characterized in that** each of the plurality of detection modules (25, 125, 225) transmits the digital data to a neighboring adjacent detection module, and at least one of the plurality of detection modules (25, 125, 225) transmits the digital data transmitted from the neighboring adjacent detection module to the data collection unit (23, 123, 223).

7. A radiography imaging apparatus comprising:
a radiation source (111) to generate radiation and to irradiate a target object with the radiation; and
a radiation detection unit (120) comprising a plurality of detection modules to detect radiation having passed through the target object to convert the radiation into an electrical signal and to convert the electrical signal into a digital signal to provide digital data and a data collection unit (23, 123, 223) to collect the digital data from the plurality of detection modules (25, 125, 225),
**characterized in that** each of the plurality of detection modules transmits the digital data to the data collection unit (23, 123, 223) using a wireless communication method.

8. The radiography imaging apparatus according to claim 6, **characterized in that**:
the data collection unit (23, 123, 223) is formed on a back plane (BP) board of a frame with the plurality of detection modules (25, 125, 225) installed thereon; and
the plurality of detection modules (25, 125, 225) receives power through a power cable (24) installed in the frame.

9. A radiation detection unit comprising:
a plurality of detection modules (25, 125, 225) to detect radiation having passed through the target object to convert the radiation into an electrical signal and to convert the electrical signal into a digital signal to provide digital data; and
a data collection unit (23, 123, 223) to collect the digital data from the plurality of detection,
**characterized in that** each of the plurality of detection modules (25, 125, 225) transmits the digital data to the data collection unit (23, 132, 223) using a wireless communication method.

10. A method of controlling a radiography imaging apparatus comprising a plurality of detection modules (25, 125, 225) to detect radiation and a data collection unit (23, 123, 223) to receive data from the plurality of detection modules, the method comprising:
irradiating a target object with radiation (511);
using a plurality of detection modules to detect radiation (having passed through the target object and converting the radiation into an electrical signal (512);
converting the electrical signal into a digital signal to provide digital data (513);
transmitting the digital data to a neighboring detection module in a single direction (514); and
transmitting the digital data transmitted from the neighboring detection module to the data collection unit (516) by at least one of the detection modules.

11. The method according to claim 10, **characterized in that** the data collection unit is formed on a back plane (BP) board of a frame with the plurality of detection modules installed thereon.

12. The method according to claim 11, **characterized in that** the transmitting of the digital data to the neighboring detection module comprises sequentially and cumulatively transmitting the digital data to the neighboring detection module in the single direction using a wired communication method through a cable installed in the frame.

13. The method according to claim 11, **characterized in that** the transmitting of the digital data to the neighboring detection module includes sequentially and cumulatively transmitting the digital data to the neighboring detection in the single direction using a wireless communication method.

14. A method of controlling a radiography imaging apparatus comprising a plurality of detection modules to detect radiation and a data collection unit to receive data from the plurality of detection modules, the method comprising:
irradiating a target object with radiation (521);
detecting by the plurality of detection modules radiation having passed through the target object and converting the radiation into an electrical signal (522);
converting by the plurality of detection modules the electrical signal into a digital signal to acquire digital data (523); and
transmitting the digital data acquired from each of the plurality of detection modules to the data collection unit using a wireless communication method (524).

15. The method according to claim 14, **characterized in that** the data collection unit is formed on a back plane (BP) board of a frame with the plurality of detection modules installed thereon.
